# EUROPÄISCHE PATENTSCHRIFT

(11) **EP 1 335 778 B1**
(45) Veröffentlichungstag und Bekanntmachung des Hinweises auf die Patenterteilung: **04.05.2005**
(21) Anmeldenummer: 01997330.4
(22) Anmeldetag: 21.11.2001
(51) Int. Cl.: A61N 5/10, G21K 5/04

(54) **VORRICHTUNG ZUR ANPASSUNG EINER IONENSTRAHLFLECKGRÖssE IN DER TUMORBESTRAHLUNG**
DEVICE FOR ADAPTING THE SIZE OF AN ION BEAM SPOT IN THE DOMAIN OF TUMOR IRRADIATION
DISPOSITIF POUR ADAPTER UNE GRANDEUR DE SPOT DE FAISCEAU IONIQUE DANS L'IRRADIATION DE TUMEURS

(30) Priorität: 21.11.2000 DE 10057824
(43) Veröffentlichungstag der Anmeldung: 20.08.2003
(73) Patentinhaber: Gesellschaft für Schwerionenforschung mbH, 64291 Darmstadt (DE)
(72) Erfinder: HABERER, Thomas, 64291 Darmstadt (DE); OTT, Wolfgang, 64291 Darmstadt (DE)
(74) Vertreter: Boeters, Hans Dietrich, Dr.
(86) Internationale Anmeldenummer: PCT/EP2001/013571
(87) Internationale Veröffentlichungsnummer: WO 2002/041948

(56) Entgegenhaltungen:
- EP-A- 0 986 070
- EP-A- 1 041 579
- EP-A- 1 045 399
- DE-A- 19 835 209

## Beschreibung

Die Erfindung betrifft eine Vorrichtung zur Anpassung einer Ionenstrahlfleckgröße in der Tumorbestrahlung gemäß dem Oberbegriff des Anspruchs 1.

Aus dem Artikel von Th. Haberer, W. Becher, D. Schardt und G. Kraft "Magnetic scanning system for heavy ion therapy", erschienen in Nuclear Instruments and Methods in Physics Research, A330 (1993), Seiten 206 - 305, ist eine Vorrichtung entsprechend dem Oberbegriff des Anspruchs 1 bekannt und ein intensitätsgesteuertes Rasterscanverfahren. Weiterhin ist aus der Patentanmeldung DE 198 35 209.3 "Vorrichtung und Verfahren zum Steuern einer Bestrahlungseinrichtung" eine Vorrichtung und ein Verfahren bekannt, daß auf einem Kontrollsystem basiert, das eine sichere Abtastung eines Tumorvolumens eines Patienten mit Hilfe eines Rasterscanverfahrens ermöglicht. Diese Vorrichtung hat jedoch den Nachteil, daß die Ionenstrahlfleckgröße während und zwischen den Bestrahlungspunkten nicht ohne großen Aufwand einstellbar ist, so daß die Ionenstrahlfleckgröße während eines jeden Schnittes durch das Tumorvolumen gleich breit bleibt und damit insbesondere im Randbereich keine scharfen Konturen zuläßt.

Aufgabe einer Weiterentwicklung dieses Kontrollsystems ist es, eine Erhöhung der geometrischen Präzision der Dosisapplikation und eine deutliche Steigerung der Robustheit des Verfahrens gegenüber Strahlpositionsschwankungen insbesondere auch im Hinblick auf den künftigen Einsatz von drehbaren Strahlführungen (Gantrys) mit integrierter Rasterscantechnik zu erreichen.

Die Größe der unvermeidbaren Strahlpositionsschwankungen aufgrund erschwerter ionenoptischer Bedingungen nimmt bei den drehbaren Strahlführungen (Gantrys) zu. Selbst wenn die Intensitäten des Therapiestrahls zwischen Maximalwert und Mittelwert um den Faktor 30 schwanken, soll und muß die Strahlpositionsschwankung des vom Beschleuniger gelieferten Therapiestrahls im Bereich von ±2 mm liegen. Somit ist es auch Aufgabe der Erfindung, eine präzise Bestrahlungsplanung umzusetzen, so daß die aus der Gesamtbestrahlung resultierende Dosisverteilung im Mittel um weniger als 5 % von der geplanten Dosisverteilung abweicht.

Diese Aufgabe wird mit dem Gegenstand des unabhängigen Anspruchs gelöst. Merkmale vorteilhafter Weiterbildungen der Erfindung ergeben sich aus den abhängigen Ansprüchen.

Erfindungsgemäß weist die Vorrichtung zur Anpassung einer Ionenstrahlfleckgröße in der Tumorbestrahlung eine Rasterscaneinrichtung aus Rasterscanmagneten zur Rasterscannung des Ionenstrahls auf. Ferner sind Ionenstrahlfleckgröße bestimmende Quadrupolmagnete unmittelbar vor dem Rasterscanmagneten vorgesehen. Das Quadrupoldoublett der strahlgrößebestimmenden Quadrupolmagnete wird erfindungsgemäß von zwei Magnetstromnetzgeräten versorgt.

Bei einer bisher verwendeten Vorrichtung können während der Extraktion des Therapiestrahls aus einem geeigneten Beschleuniger wie einem Synchrotron zeitliche Veränderungen von ionenoptischen Parametern im Beschleuniger oder der nachfolgenden Strahlführung sowohl Strahlpositionsschwankungen als auch zeitliche Veränderungen der Strahlfleckgrößen verursachen. Während das Teilproblem der Strahlpositionsschwankungen zwischenzeitlich äußerst effektiv gelöst wurde, gibt es bisher kein Verfahren, die Strahlfleckgrößenschwankungen zu vermindern oder zu beherrschen.

Aus EP 1 041 579 ist eine Vorrichtung bekannt, wobei die lonenstrahlfleckgröße steuerbar ist mit Hilfe von Quadrupolmagneten. Dieses Dokument offenbart den Oberbegriff des Anspruchs 1. Aus EP 1 045 399 ist bekannt, Stromkorrekturwerte bereitzustellen für Magnetstrom-Netzgeräte zur Korrektur der Strahlposition.

Ziel einer Tumorionenbestrahlung ist es jedoch, möglichst exakte Teilchenbelegungen zu erzeugen, das heißt, innerhalb des Zielvolumens sollen die Abweichungen von der geplanten Dosisverteilung minimal werden, sind die Strahlbreitenvariationen nur in einem bestimmten Maß tolerabel, da sonst das geometrische Muster der Strahlpositionen, welches im Rahmen einer Bestrahlungsplanung vorher festgelegt wurde, nur für einen definierten Bereich der Strahlbreite ausreichend exakte Bestrahlungsergebnisse liefern kann.

Deshalb weist erfindungsgemäß die Vorrichtung eine Regelschleife auf, die Stromkorrekturwerte unter Vergleich von Soll- und Istwerten einer aktuellen Strahlenausdehnung für zwei Magnetstromnetzgeräte des unmittelbar vor den Rasterscanmagneten angeordneten Quadrupoldoubletts und eine definierte Homogenisierung und/oder eine definierte Variation der Strahlfleckgröße, während der Strahlenextraktion und/oder von Meßzyklus zu Meßzyklus und/oder von Strahlposition zu Strahlposition bereitstellt.

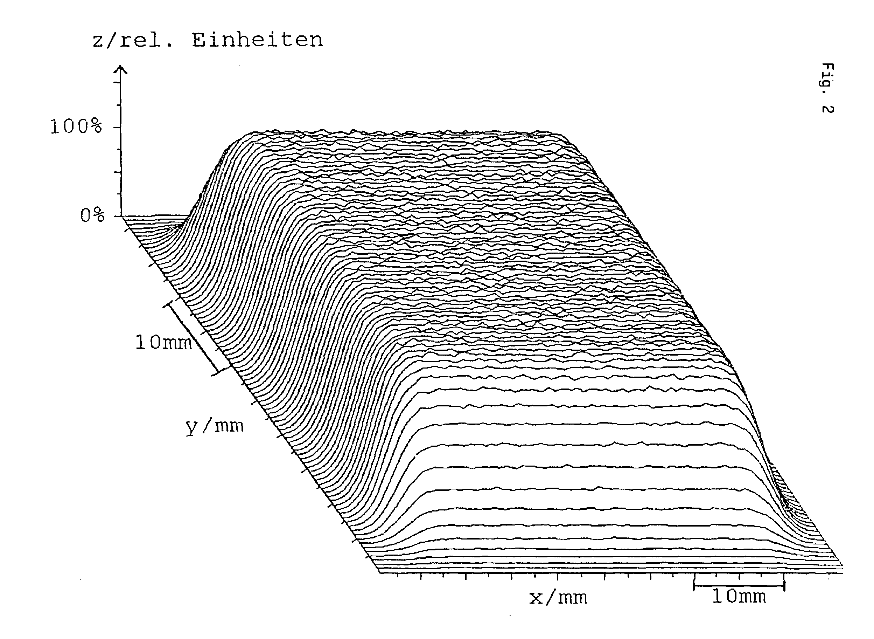

Die erfindungsgemäße Lösung hat den Vorteil, daß ein sinnvolles Verhältnis von Strahlprofilbreite zu Strahlpositionsabstand während der Bestrahlung eingehalten werden kann, und es ergeben sich damit homogene Dosisverteilungen, die den medizinischen Anforderungen genügen, wie sie in Figur 2 gezeigt werden.

Um eine derart homogene Strahlenverteilung zu erreichen, weist vorzugsweise die Vorrichtung eine Echtzeitsoftware auf zur Berechnung der Istwerte der Ionenstrahlbreite aus Detektorrohdaten. Darüber hinaus weist die Vorrichtung einen ortsempfindlichen Detektor zur Erfassung der Ionenstrahlbreite und zur Erzeugung von Detektorrohdaten der Ionenstrahlbreite auf. Ein Vorteil dieser Ausführungsform des Erfindung besteht darin, die Erzeugung sehr steiler Dosisgradienten am Rand des zu bestrahlenden Volumens durch die Variation der Ionenstrahlbreite zum Rand hin zu ermöglichen.

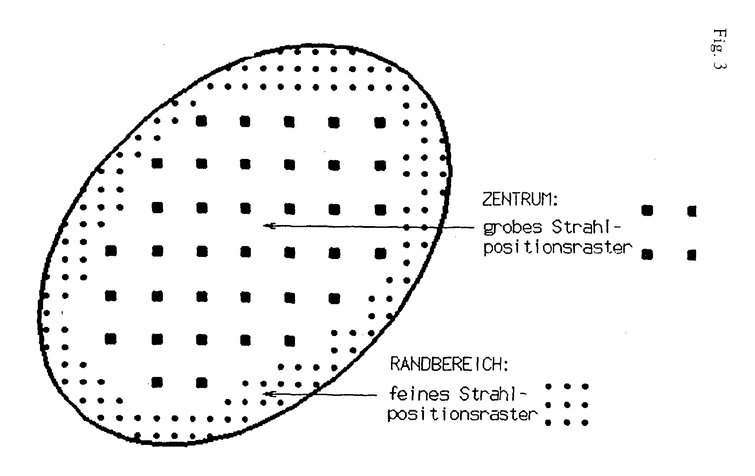

Die Strahlfleckgröße wird für einen steilen Randabfall zum Rand hin minimiert, so daß der Bereich dieses Dosisabfalls skaliert mit der Halbwertsbreite des Therapiestrahls möglich ist. Mit dieser Vorrichtung zur Regelung der Strahlfleckgröße ist der Vorteil verbunden, daß die Bestrahlungsdauer für den Patienten im Bestrahlungsraum in immobilisierter Lage des Patienten minimiert werden kann, indem eine große Ionenstrahlfleckgröße in weiten Bereichen des Tumorvolumens durchführbar ist und nur zum Rand hin, - um eine präzisere Nachzeichnung des Randes zu erhalten, - die Ionenstrahlfleckgröße vermindert und die Strahlpositionen pro Flächeneinheit verdichtet werden, wie es in Figur 3 gezeigt wird.

Die Dauer einer Bestrahlung und damit auch der Patientendurchsatz einer Anlage wird durch die verminderte Dichte der Strahlpositionen im Volumen des Tumors vermindert, da mit abnehmender Dichte der Strahlpositionen auch die Dauer der Bestrahlung abnimmt. Somit wird vorteilhaft eine Verringerung der Bestrahlungsdauer durch die Möglichkeit der Strahlfleckgrößeneinstellung erreicht, weil weniger Strahlpositionen in größerem Abstand im Volumeninneren des Tumors geplant werden können. Um jedoch andererseits eine ausreichende Qualität der Teilchenbelegungen über dem Querschnitt des Tumors zu gewährleisten, wird bei gegebenem Strahlpositionsabstand eine Mindestbreite des Strahlflecks gefordert. Diese wiederum wird durch die erfindungsgemäße Vorrichtung gewährleistet.

In einer weiteren bevorzugten Ausführungsform der Vorrichtung weist diese Steuer- und Auslesemodule und zugehörige Datenverbindungen auf, um Informationen über den Istwert von Ionenstrahlbreiten an ein Speicher-, Steuer- und Auslesemodul zu schicken und die Meßdaten zu speichern. Diese Ausführungsform der Erfindung hat den Vorteil, daß durch Zusammenwirken von Steuer- und Auslesemodul mit den Magnetstromnetzgeräten des Quadrupoldoubletts der Ionenstrahlpunktgröße bestimmenden Quadrupolmagnete eine zügige Variation der Ionenstrahlfleckgröße gewährleistet werden kann.

In einer weiteren Ausführungsform der Erfindung weist das Auslesemodul eine Anzahl freier Schnittstellen und eine Rechenleistung auf, über welche die Nachführung der Ionenstrahlbreite ausführbar ist. Somit wird vorteilhaft die aus medizintechnischer Sicht verständliche Forderung eines steilen und präzisen Randabfalls bei minimaler Bestrahlungsdauer bezüglich der Bestrahlungsfleckgröße erreicht, was im derzeitigen Stand der Technik aufgrund der starren Ionenstrahlfleckgröße nicht erreichbar ist. Mit der Nachführbarkeit und Einstellbarkeit der Strahlfleckgröße und der Strahlfleckposition ergibt sich vorteilhaft eine dynamische Anpassung während der Bestrahlung eines Tumorvolumens, wodurch auch dem Bestrahlungsplaner eine erhöhte Flexibilität in der Festlegung des Strahlpositionsmusters bereitgestellt wird und die Qualität der Dosisverteilung weiter gesteigert werden kann.

In einer alternativen Ausführungsform der Vorrichtung wird ein um ein Steuer- und Auslesemodul erweitertes Kontrollsystem bereitgestellt, wobei das zusätzliche Steuer- und Auslesemodul ausschließlich die Funktion der Ionenstrahlbreitenregelung ausführen kann. Mit dieser Ausführungsform wird die schnelle Adjustierung in Zeitintervallen möglich, die wesentlich kürzer als typische Beschleunigerzykluszeiten sind, erreicht.

In einer weiteren bevorzugten Ausführungsform der Erfindung weist die Vorrichtung eine Regelschleife auf, die von einer Vieldrahtionisationskammer mit angeschlossenem Steuer- und Auslesemodul ausgeht und zu einem Steuer- und Auslesemodul führt, das Meßdaten zur graphischen Darstellung bereitstellt, und zwei Magnetstromgeräte zur horizontalen und vertikalen Fokussierung des Quadrupoldoubletts unmittelbar vor dem Rasterscanmagneten ansteuert. Damit werden vorteilhaft korrigierte Feldeinstellungen erzeugt, welche den Fokussierzustand der Anlage verbessern. Die Regelung kann sowohl die Aufrechterhaltung einer konstanten Ionenstrahlfleckgröße während des Scanprozesses als auch die definierte Variation der Ionenstrahlfleckgröße gemäß dem Vorgang einer Bestrahlungsplanung in vorteilhafter Weise sicherstellen. Dabei wird die aus der Ortsmessung des Rasterscansystems verfügbare Information zum zeitlichen Verlauf der Strahlbreite kombiniert mit der Fähigkeit des Kontrollsystems, Magnetstromnetzgeräte von Ionenstrahlfleckgröße beeinflussenden Magneten wie z.B. Quadrupolmagneten in schneller zeitlicher Abfolge mit neuen Sollwerten zu beaufschlagen, so daß aus dem aktuellen Vergleich von Soll- und Istwert der aktuellen Strahlausdehnung Stromkorrekturwerte für beide Magnetstromnetzgeräte eines Quadrupoldoubletts unmittelbar vor dem Rasterscanmagneten errechnet werden können.

Ein Verfahren zur Anpassung einer Ionenstrahlfleckgröße in der Tumorbehandlung wird durch folgende Verfahrensschritte gekennzeichnet:
- dynamisches Anpassen der Ionenstrahlfleckgröße in Echtzeit unter
- Beaufschlagen des Ionenstrahlfleckgröße bestimmenden Quadrupolmagnetes mit Sollwerten für unterschiedliche Ionenstrahlfleckgröße innerhalb eines Beschleunigungszyklus und/oder zwischen zwei aufeinanderfolgenden Beschleunigungszyklen und/oder zwischen benachbarten Strahlpositionen mittels einstellbarer Frequenz,
- Gewinnung von Stromkorrekturwerten unter Vergleich von Soll- und Istwerten einer aktuellen Strahlausdehnung für zwei Magnetstromnetzgeräte des Quadrupoldoubletts der Ionenstrahlfleckgröße bestimmenden Quadrupolmagneten zur definierten Homogenisierung und/oder zur definierten Variation der Ionenstrahlfleckgröße, während der Strahlextraktion und/oder von Meßzyklus zu Meßzyklus und/oder von Strahlposition zu Strahlposition,
- Bestrahlen eines Tumorgewebes mit einem dichteren Ionenstrahlpositionsraster bei gleichzeitig geringerer Ionenstrahlfleckgröße im Randbereich als im Volumenbereich des Tumors.

Somit verbessert ein Verfahren zur Anwendung der erfindungsgemäßen Vorrichtung das früher beschriebene Rasterscanverfahren in mehreren Aspekten. Durch die Möglichkeit, die Ionenstrahlfleckgröße in Echtzeit einzustellen, kann die Qualität der vom Rasterscanner erzeugten Teilchenbelegungen gesteigert werden, indem die aktuelle Strahlbreite aktiv an die Vorgaben der Bestrahlungsplanung durch ein Regelungsverfahren angepaßt wird. Hierdurch kann die geometrische Dichte der Strahlpositionen gegenüber dem bisherigen Bestrahlungsmodus verringert werden, zumal bisher eine Reserve vorbehalten werden mußte, um die stets vorkommenden Schwankungen der Strahlbreite auszugleichen. Das erfindungsgemäße Regelungsverfahren, welches diese Schwankungen minimiert, eröffnet die Möglichkeit, eine geringere Zahl an Strahlpositionen zu planen, dies führt zu kürzeren Bestrahlungszeiten und einem höheren Patientendurchsatz.

Bei der erfindungsgemäßen Vorrichtung wird die Strahlhalbwertsbreite dem Ionenstrahlpositionsraster in der Weise angepaßt, daß bei feinem Ionenstrahlpositionsraster im Randbereich eine geringere Strahlhalbwertsbreite eingestellt wird als im Volumenbereich des Tumors mit einem groben Ionenstrahlpositionsmuster. Damit lassen sich vorteilhaft im Randbereich präzisere Abgrenzungen zwischen Tumorgewebe und gesundem Gewebe erzielen, da gleichzeitig die Strahlhalbwertsbreite geringer und damit schärfer eingestellt wird als im Volumenbereich des Tumors. Durch die größere Strahlhalbwertsbreite im Volumenbereich des Tumors mit einem groben Ionenstrahlmuster kann vorteilhaft die Gesamtzahl der Strahlpositionen pro Isoenergieschnitt, das heißt pro Bestrahlungsebene, vermindert werden und damit die Strahlbehandlungszeit verringert werden. Andererseits wird der Dosisgradient am Rand des Tumorgewebes sehr steil und somit eine präzise Abgrenzung zwischen Tumorgewebe und gesundem Gewebe sichergestellt.

Ein bevorzugtes Durchführungsbeispiel des Verfahrens zur Verwendung der erfindungsgemäßen Vorrichtung sieht vor, daß für jeden Meßzyklus eine Echtzeitsoftware in einem Steuer- und Auslesemodul einer Vieldrahtionisationskammer den Istwert der Ionenstrahlbreite aus den Detektorrohdaten der Vieldrahtionisationskammer errechnet. In diesem Fall wird ein Verfahren vorzugsweise durchgeführt, bei dem die Strahlhalbwertsbreite von Meßzyklus zu Meßzyklus variiert und eingestellt wird, wobei die Strahlhalbwertsbreitenberechnung über die Erfassung von Detektorrohdaten einer Vieldrahtionisationskammer erfolgt. Eine derartige Echtzeitsoftware hat zwischen Meßzyklus zu Meßzyklus und/oder zwischen benachbarten Strahlpositionen genügend Zeit, um die entsprechenden Einstelldaten neu und angepaßt zu berechnen.

In einem weiteren bevorzugten Durchführungsbeispiel des Verfahrens zur Verwendung der erfindungsgemäßen Vorrichtung werden Informationen der Strahlbreite an ein Modul zum Speichern der Meßdaten sowie zum Steuern und Auslesen geliefert. Dieses Modul kann einerseits dafür dienen, daß die Rohdetektordaten zunächst gespeichert und von Strahlposition zu Strahlposition oder von Meßzyklus zu Meßzyklus neu korreliert werden.

In einem weiteren Durchführungsbeispiel des Verfahrens zur Verwendung der erfindungsgemäßen Vorrichtung wird ein Modul zum Speichern, Steuern und Auslesen der Istwerte der Ionenstrahlfleckgröße mit der Information über den Sollwert der Ionenstrahlfleckgröße aus einem Bestrahlungsplan in Echtzeit verglichen. Mit einem derartigen Modul kann die Ionenstrahlfleckgröße nicht nur zwischen Meßzyklus und Meßzyklus und/oder zwischen Strahlposition zu Strahlposition erfaßt und variiert werden, sondern noch während und innerhalb einer Strahlextraktion variiert werden. Dazu wird in einem weiteren Durchführungsbeispiel des Verfahrens ein Korrekturwert für Magnetnetzgeräte der Quadrupoldoublette der Ionenstrahlfleckgröße bestimmenden Quadrupolmagnete einer Hochenergiestrahlungsführung unmittelbar vor dem Rasterscanner ermittelt und entsprechend eingestellt.

Wird in einem weiteren Durchführungsbeispiel des Verfahrens zur Verwendung der erfindungsgemäßen Vorrichtung von Meßzyklus zu Meßzyklus gearbeitet, so kann ein Ortsmeßsystem von einer Ionenstrahlposition im Strahlungsplan zur nächsten Ionenstrahlposition eine Korrektur und Neueinstellung durchführen. Diese Neueinstellung wird zunächst durch ein Einstellen der Frequenz einer Nachführung des Ionenstrahl bereits in Form eines Parameters einer Echtzeitsoftware vorgegeben, welche die Korrekturwerte für die Quadrupoldoublette ermittelt und durchführt. Auch die Dämpfung der Ionenstrahlbreite ist analog zur Frequenz der Anpassung über einen Parameter einstellbar. Schließlich können Schwellenwerte für die Ionenstrahlbreite in bezug auf maximale und minimale Ionenstrahlbreite festgelegt werden, um Fehlanpassungen von vorneherein auszuschließen.

Somit betrifft die erfindungsgemäße Vorrichtung und das Verfahren dessen Anwendung eine Nachregelung der Strahlbreite in Echtzeit. Hierzu wird die aus der Ortsmessung des Rasterscansystems verfügbare Information zum zeitlichen Ablauf der Strahlbreite kombiniert mit der Fähigkeit des Kontrollsystems, Magnetstromnetzgeräte von Ionenstrahlfleckgröße beeinflussenden Magneten wie Quadrupolmagneten in schneller zeitlicher Abfolge mit neuen Sollwerten zu beaufschlagen. Hierdurch wird eine Regelschleife aufgebaut, welche es ermöglicht, aus dem Vergleich von Soll- und Istwerten der aktuellen Strahlausdehnung Stromkorrekturwerte für die beiden Magnetstromnetzgeräte des Quadrupoldoubletts unmittelbar vor den Rasterscanmagneten zu errechnen und somit korrigierte Feldeinstellungen zu erzeugen, welche den Fokussierzustand einer Ionenstrahltherapieanlage verbessern. Somit kann sowohl die Regelung für eine konstante Ionenstrahlfleckgröße sorgen und zuverlässiger während des Scanprozesses beibehalten werden als auch definierte Variationen der Ionenstrahlfleckgröße gemäß den Vorgaben einer wesentlich flexibler gestaltbaren Bestrahlungsplanung durchgeführt werden.
Figur 1 zeigt ein Beispiel für eine inhomogene Dosisverteilung bei zu geringer Strahlhalbwertsbreite.
Figur 2 zeigt ein Beispiel für eine homogene Dosisverteilung bei ausreichender Strahlhalbwertsbreite.
Figur 3 zeigt ein Ausführungsbeispiel einer Strahlpositionsverteilung, die durch Einsatz der erfindungsgemäßen Vorrichtung möglich wird.
Figur 4 zeigt eine Ausführungsform eines Bestrahlungssystems, in dem die erfindungsgemäße Vorrichtung einsetzbar ist.
Figur 5 zeigt einen Datenflußplan eines Ausführungsbeispiels der Erfindung.

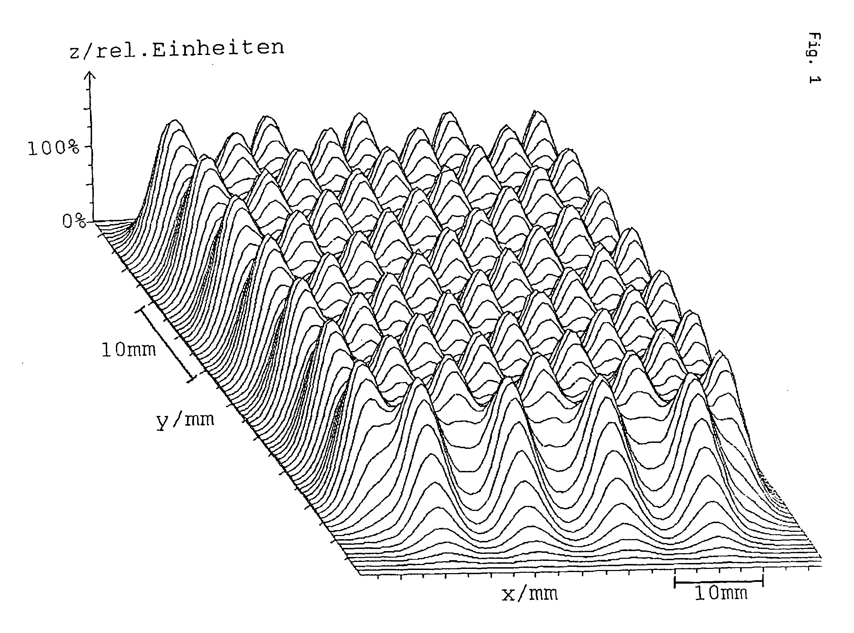

Figur 1 zeigt ein Beispiel für eine inhomogene Dosisverteilung bei zu geringer Strahlhalbwertsbreite. Dazu ist in Figur 1 ein Koordinatensystem in X-, Y- und Z-Richtung aufgepannt, wobei die Einheit für die X- und die Y-Achse Millimeter sind und in Richtung der Z-Achse die Strahlendosis aufgetragen ist. Figur 1 illustriert die Auswirkung einer nicht mit Vorgaben einer Strahlungsplanung konformen Strahlprofilbreite auf die Homogenität der Dosisverteilung. An den in Z-Richtung herausragenden Maxima von Bestrahlungsdosen ist eine für eine Tumorbestrahlung nicht vertretbare Inhomogenität zu erkennen.

Im Prinzip ist die Strahlprofilbreite im Verhältnis zu den Strahlungspositionsabständen zu schmal oder umgekehrt, die Strahlungspositionsabstände sind für die eingestellte Strahlprofilbreite zu groß. Ein wesentliches Ziel der Strahlapplikation wird somit nicht erreicht, nämlich die Erzeugung von möglichst exakter Teilchenbelegung, das heißt, innerhalb des Zielvolumens sollen die Abweichungen von der geplanten Dosisverteilung minimal sein, und somit ergibt sich für die in Figur 1 eingestellte Strahlprofilbreite eine Bestrahlungsinhomogenität, und die Vorgaben der Bestrahlungsplanung können nicht eingehalten werden.

Figur 2 zeigt ein Beispiel für eine homogene Dosisverteilung bei ausreichender Strahlhalbwertsbreite. In Figur 2 ist wieder ein X-, Y- und Z-Koordinatensystem dargestellt mit einer Millimetereinteilung in der X- und der Y-Achse sowie eine Dosisgröße in Richtung der Z-Achse. Bei dieser Ausführungsform wird ein sinnvolles Verhältnis von Strahlprofilbreite zu Strahlpositionsabstand während der Bestrahlung eingehalten, so daß sich eine homogene Dosisverteilung, die den medizinischen Anforderungen genügt, im Tumorvolumen ergibt und ein relativ steiler Abfall zum Rand der Bestrahlung erreichbar wird. Dazu muß jedoch ein wesentlich geringerer Strahlpositionsabstand und damit wesentlich mehr Strahlpositionen in Figur 2 geplant werden als in Figur 1, so daß die Behandlungszeit in Figur 2 um ein Vielfaches größer wäre als die Behandlungszeit in Figur 1. Mit Hilfe der vorliegenden Erfindung wird jedoch die Strahlbreite von Strahlposition zu Strahlposition variiert, so daß mit größer werdendem Strahlpositionsabstand die Strahlprofilbreite breiter eingestellt werden kann und zum Rand hin der Strahlpositionsabstand verringert wird bei gleichzeitiger Verringerung der Strahlprofilbreite.

Somit können durch die erfindungsgemäße Vorrichtung wesentlich homogenisiertere Dosisverteilungen wie sie Fig. 2 zeigt erreicht werden und gleichzeitig die Anzahl der Strahlpositionen pro Isoschnitt durch ein Tumorgewebe vermindert werden, und zum Rand des Tumorgewebes hin der Strahlpositionsabstand vermindert und gleichzeitig die Strahlprofilbreite verkleinert werden, wie es Fig.3 zeigt, so daß ein steiler Randabfall und eine präzisere Abgrenzung zum gesunden Gewebe möglich wird.

Figur 3 zeigt die Strahlpositionsverteilung, die durch Einsatz der erfindungsgemäßen Vorrichtung erreicht wird. Die erfindungsgemäße Vorrichtung erlaubt eine Anpassung einer Ionenstrahlfleckgröße in der Tumorbestrahlung mit einer Rasterscaneinrichtung aus Rasterscanmagneten zur Rasterscannung des Ionenstrahls, mit Ionenstrahlfleckgröße bestimmenden Quadrupolmagneten, die unmittelbar vor den Rasterscanmagneten angeordnet sind, mit zwei Magnetstromnetzgeräten des Quadrupoldoubletts der Ionenstrahlfleckgröße bestimmenden Quadrupolmagnete, wobei die Vorrichtung eine Regelschleife aufweist, die Stromkorrekturwerte bereitstellt unter Vergleich von Soll- und Istwerten einer aktuellen Strahlenausdehnung für zwei Magnetstromnetzgeräte des unmittelbar vor den Rasterscanmagneten angeordneten Quadrupoldoubletts und eine definierte Homogenisierung und/oder eine definierte Variation der Ionenstrahlfleckgröße, während der Strahlextraktion und/oder von Meßzyklus zu Meßzyklus und/oder von Strahlposition zu Strahlposition ermöglicht.

Figur 3 zeigt dazu ein Tumorgewebe 1 umgeben von einem gesunden Gewebe 2 und einen scharf abgegrenzten Rand 3, der das Grenzgewebe zwischen Tumorgewebe 1 und gesundem Gewebe 2 darstellt. Mit der oben erwähnten erfindungsgemäßen Vorrichtung wird die Strahlprofilbreite im Zentrum 4 des Tumorgewebes größer eingestellt als im Randbereich 9 des Tumorgewebes 1. Dafür wird im Randbereich 9 ein feines Strahlpositionsraster vorgesehen, das in dieser Ausführungsform eine vierfache Flächendichte im Randbereich 9 gegenüber der Flächendichte im Zentrum 4 aufweist.

Aufgrund der erfindungsgemäßen Strahlbreitenregelung, die unmittelbar auf die vor den Rasterscanmagneten angeordneten Quadrupoldoubletts, sowohl in horizontaler als auch in vertikaler Richtung einwirkt, ist es möglich, trotz des groben Strahlpositionsrasters im Zentrum 4 des Tumorgewebes eine Homogenisierung der Dosisverteilung zu realisieren und gleichzeitig eine homogene Dosisverteilung auch im Randbereich 9 zu erreichen mit wesentlich präziserer Abgrenzung des Randbereichs des Tumorgewebes 1 gegenüber dem gesunden Gewebe 2 aufgrund der Anpassung von Strahlposition zu Strahlposition der Ionenstrahlfleckgröße während der Bestrahlung. Diese Anpassung kann nicht nur von Strahlposition zu Strahlposition durchgeführt werden, sondern auch von Meßzyklus zu Meßzyklus und zur Stabilisierung der Dosisverteilung auch während der Strahlextraktion erfolgen. Dazu ist eine zusätzliche Regelschleife zwischen einem Steuerungs- und Auslesemodul und Magnetnetzgeräten für horizontale und vertikale Quadrupole vorgesehen, die unmittelbar vor dem Scanmagneten angeordnet sind.

Somit wird zur Bestrahlung diese Isoenergieschnittes im Randbereich ein schmaler Ionenstrahlfleck auf einem engen Strahlpositionsraster und im Zentrum ein großer Strahlfleck auf einem groben Strahlpositionsraster durchgeführt. Hierdurch kann vorteilhaft die Gesamtzahl der Strahlpositionen pro Isoenergiequerschnitt und damit die Bestrahlungsdauer deutlich herabgesenkt werden, und der Dosisgradient am Rand sehr steil bei der Vorgabe der Bestrahlungsplanung gewählt werden.

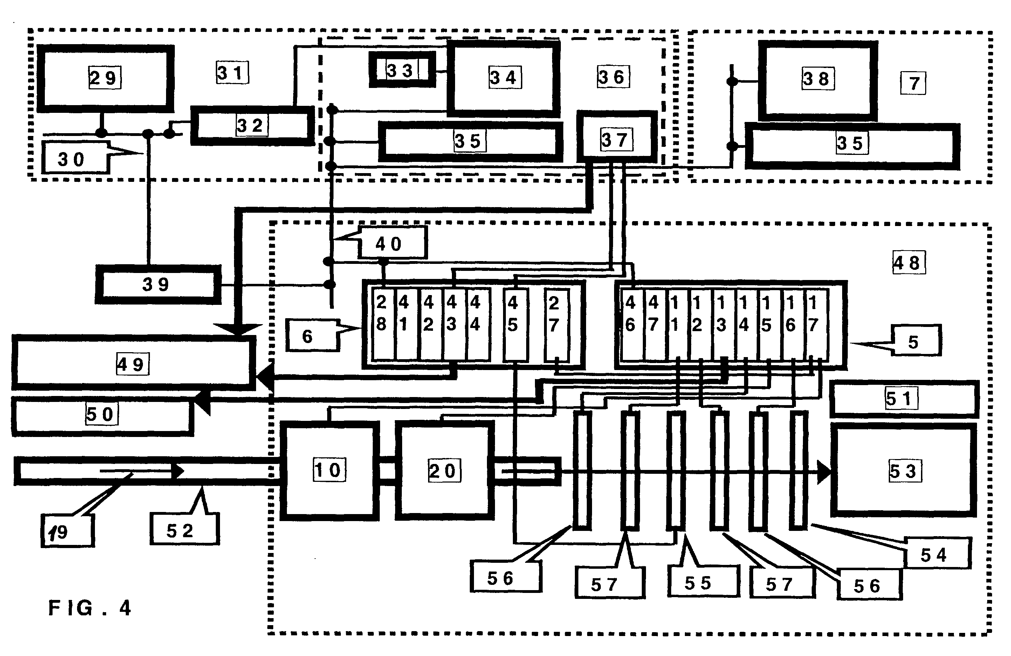

Figur 4 zeigt eine Ausführungsform eines Bestrahlungssystems, in dem die erfindungsgemäße Vorrichtung einsetzbar ist. Dabei wird die Steuerung und Überwachung des Bestrahlungssystems durch ein komplexes elektronisches System gewährleistet.

Das Steuerungs- und Überwachungssystem setzt sich aus drei Ebenen zusammen, nämlich einer Ablaufsteuerung 5, einer Systemkontrolle 6 und einer Bedienerführung 7. Diese arbeiten unabhängig voneinander. Verteilt über alle drei Ebenen ist ein Sicherheitssystem 8 realisiert, welches eine sofortige Abschaltung des Strahls im Falle einer Störung im System gewährleistet.

Die Ablaufsteuerung 5 erlaubt einen Zugriff von der Bedienerführung 7 nur bei der Initialisierung beim Start und beim Notstop. Während der Bestrahlung agiert die Ablaufsteuerung 5 automatisch. Sie erfüllt neben den Steuerungsaufgaben auch Sicherheitsfunktionen, indem die gemessenen Daten mit den Vorgaben des Bestrahlungsplanes verglichen werden und bei Abweichung oberhalb festgelegter Grenzen zur Abschaltung des Strahls führen.

Die Systemkontrolle 6 erlaubt die Einstellung der Betriebsparameter, zum Beispiel der Detektorspannung. Des weiteren überwacht die Systemkontrolle 6 "langsam" ablaufende Vorgänge durch Auslesen einer Vielzahl von Systemzuständen und schaltet gegebenenfalls den Strahl ab.

Die Bedienerführung 7 ermöglicht dem Bediener die Interaktion mit dem Steuerungs- und Überwachungssystem. Von hier aus werden Bestrahlungspläne in die Ablaufsteuerung 5 geladen, Bestrahlungen gestartet, gestoppt oder unterbrochen, Bedieneraktionen und Systemparameter protokolliert, der Bestrahlungsvorgang und der Systemzustand anhand der Meßdaten visualisiert und die gemessenen Daten zu Dokumentszenen archiviert.

Das Steuerungs- und Überwachungssystem ist als VME-Umgebung realisiert und weist Bedienungseinrichtungen wie Ein-/Ausgabegeräte (Terminals) sowie eine Rechenanlage bestehend aus mehreren Einzelrechnern mit den üblichen Peripheriegeräten auf. Die Einrichtungen zur Überwachung des Strahls bezüglich Ort, Breite und Intensität sowie die Einrichtung zur Anforderung und Ablenkung des Strahls sind über Busleitungen an die VME-Umgebung gekoppelt.

Das von der Ablaufsteuerung 5 unabhängig arbeitende Sicherheitssystem überwacht den Betragsstrahlungsvorgang während der gesamten Dauer der Bestrahlung. Es unterbricht den Bestrahlungsvorgang selbsttätig, wenn infolge einer Fehlfunktion die Ablenkung des Strahles fehlerhaft ist oder die Teilchenzahl, für einen Punkt für eine Schicht oder die insgesamt aufgebrachte Teilchenzahl überschritten wird. Hierbei kann die Ursache einer Fehlfunktion bereits in der Strahlerzeugung liegen oder in der Ablaufsteuerung 5 begründet sein, wobei die Ablaufsteuerung 5 jedoch Selbstkontrollmittel zur Unterbrechung des Bestrahlungsvorgangs aufweist.

Die Ablaufsteuerung 5 weist Schaltungsmodule (Steuerungs- und Auslesemodule 11 - 17) auf, die durch einen gemeinsamen Systembus mit den Bedienungseinrichtungen verbunden sind. Der Systembus ist als VME-Bus ausgebildet.

Jedes der Steuerungsmodule 11 bis 17 ist jeweils durch einen separaten Gerätebus mit einer Meßeinrichtung (wie Ionisationskammer, Vieldrahtkammer usw.) bzw. mit einer externen Speichervorrichtung 27 verbunden. Die Gerätebusse sind von dem Systembus unabhängig. Das mit Figur 4 gezeigte Blockschaltbild gehört somit zu einem Kontrollsystem für eine Ionenstrahltherapieanlage.

Das Kontrollsystem für eine Ionenstrahltherapieanlage besteht somit im wesentlichen aus einem technischen Kontrollraum (TKR), in dem auf einer Beschleuniger-Operating-Konsole alle Beschleunigerdaten des Ethernet auflaufen und Ethernet-Router-Daten an die nächste größere Einheit des Kontrollsystems für eine Ionenstrahltherapieanlage der techischen Operatingkonsole in der Therapie selbst weitergeleitet wird. Das zentrale Gerät dieser technischen Operating-Konsole ist der Therapie-Operating-Rechner (TORT), der einen Barcodeleser (BCL) aufweist und der über das Therapie-Ethernet mit dem Bedienungselement der Terminals in Verbindung steht. Die technische Operating-Konsole im Therapiebereich verfügt über eine medizinische Bedienkonsole (MBDK), die mit einem Therapiebereich (Cave M) in Verbindung steht und über eine unmittelbare Verbindung zum Auslösen eines Strahlabbruchs des Beschleunigers verfügt. Zum Strahlabbruch wird ein Resonanzquadrupol (S02IQ1E) für die langsame Extraktion des Strahls über sein Netzgerät über eine Interlockeinheit in dem Bussystem des Therapiekontrollsystems auf Null gesetzt. Ein Ablenkdipolmagnet (TH3MU1) der Strahlführung zum Therapiemeßplatz wird ebenfalls zum Strahl- oder Extraktionsabbruch in einem Fehlerfall durch die Interlockeinheit (ILE) in dem Bussystem (VME) des Therapiekontrollsystems auf Null gesetzt.

Für die Systemkontrolle (VMESK) an sich wirken mehrere Mikroprozessoren auf einem Bussystem-Verbindungsrahmen (VME-Crate) zusammen. Dazu gehört neben dem oben erwähnten und in Figur 4 dargestellten Datenspeicher (ODS) für eine Online-Anzeige ein Intensitätsmonitor (IMON), der unter anderem mit einer Ionisationskammer und der Ausleseelektronik zur Überwachung der Gesamtteilchenzahl zusammenwirkt. Darüber hinaus befindet sich in der Systemkontrolle eine Trottmann-Schaltungseinheit (TME) zur Überwachung der Funktionsfähigkeit der Prozessoren. Neben der bereits erwähnten Interlockeinheit (ILE) und einem Kontrollbusadapter (KBA) verfügt die Systemkontrolle über ein Analog-/Digitalmodul (ADIO) und einen Systemkontrollrechner (SKR) in dem Bussystem (VME) der Systemkontrolle.

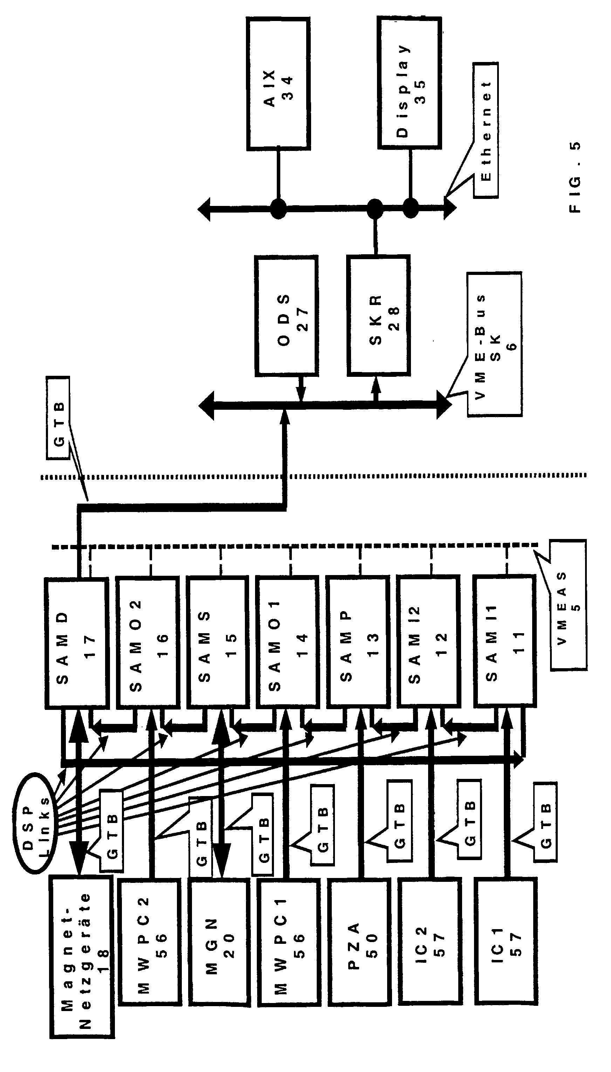

Die Komponenten der Ablaufsteuerung (VMEAS) sind identisch mit den Komponenten des in Figur 5 gezeigten Datenflußplans, wobei die Ablaufsteuerung in dem in Figur 4 gezeigten Kontrollsystem zusätzlich ein digitales Eingangs-/Ausgangsmodul (DIO) und einen Ablaufsteuerungsrechner (ASR) aufweist. Im Therapiebereich (Cave M) befindet sich ein Positron-Emitter-Tomograph (PET) zur räumlichen Bestimmung der Teilchenreichweite durch Positronen emittierende Strahlung, mit der die Bestrahlungswirkung auf einen Patienten auf der Patientenliege nachgewiesen werden kann.

Die Ionenstrahlführung in den Therapiebereich (Cave M) hinein wird im unteren Bereich der Figur 4 prinzipiell dargestellt, wobei der Strahl für die örtliche Abtastung durch Scannermagnete für X und Y geführt wird, die mit Hilfe von Magnetstromnetzgeräten (MGM) des Rasterscanners den Strahl horizontal (X) und (Y) ablenken.

Nach dem Verlassen des Strahls nach dem letzten nichtgezeigten Ablenkungsmagneten wird der Strahl noch vor der Patientenliege durch eine Mehrzahl von Detektoren geführt, wobei eine Regelschleife über einen ersten ortsempfindlichen Detektor (MWPC1) auf die Magnetstromnetzgeräte (MGM) des Rasterscanners für dessen Scannermagnete wirkt, so daß die Strahlposition durch Nachführung von Strahlposition zu Strahlposition korrigiert werden kann oder durch Nachführen von Meßzyklus zu Meßzyklus oder innerhalb einer einzelnen Strahlposition während der Strahlextraktion korrigiert werden kann.

Mit der erfindungsgemäßen Vorrichtung wird nun zusätzlich eine Regelschleife für das Quadrupoldoublett unmittelbar vor den Rasterscanmagneten für die horizontale (X) und die vertikale (Y) Richtung eingesetzt, wobei die Magnetstromnetzgeräte 18 des Quadrupoldoubletts für die horizontale und vertikale Richtung entsprechend angesteuert werden, indem eine Regelschleife Stromkorrekturwerte unter Vergleich von Soll- und Istwerten einer aktuellen Strahlenausdehnung für zwei Magnetstromnetzgeräte 18 des unmittelbar vor dem Rasterscanner angeordneten Quadrupoldoubletts bereitstellt. Somit stellt dieser Hardwareüberblick eine weitere vorteilhafte Weiterentwicklung des Systems dar, wobei die wesentliche Neuerung in dem erfindungsgemäßen Strahlungsssystem die Hinzunahme dieser Regelschleife ausgehend von der Vieldrahtionisationskammer (MWPC1) mit angeschlossenem Steuer- und Auslesemodul (SAMO1) zum Steuer- und Auslesemodul (SAMD), welches die Meßdaten zur graphischen Darstellung bereitstellt und die Magnetstromnetzgeräte 18 des Quadrupoldoubletts unmittelbar vor den Rasterscanmagneten ansteuert, wodurch die Echtzeitnachregelung der Strahlbreite ermöglicht wird.

Figur 5 zeigt einen Datenflußplan eines Ausführungsbeispiels der Erfindung. Dabei dient der oberste Mikroprozessor in dem Ablaufsteuersystem (VMEAS) als Steuer- und Auslesemodul (SAMD) zur Online-Datenübertragung in den Datenspeicher (ODS) in der sich anschließenden und auf der rechten Seite der Bildhälfte der Figur 5 abgebildeten Systemkontrolle. Dieses Steuer- und Auslesemodul (SAMD) zur Online-Datenübertragung ist mit dem Datenspeicher (ODS) für eine Online-Anzeige über den Gerätebus verbunden, der ein differentieller Datenbus zwischen den Steuer- und Auslesemodulen und deren jeweiliger Front-End-Elektronik ist.

Der Datenspeicher (ODS) für eine Online-Anzeige liefert seine Daten entsprechend dem Datenflußplan nach Figur 5 über den Bus der Systemkontrolle (SK) und den Systemkontrollrechner in dem Bussystem (VME) zur Verbindung von Prozessoren und Datenmodulen einerseits an das Display und andererseits an das Ethernet unter Führung des Betriebssystems (AEX) in der Systemkontrolle.

In der Ausführungsform nach Figur 5 weist das Regelsystem in der Ionenstrahltherapie mindestens eine Ionisationskammer (IC1) auf, die der Intensitätsmessung des Ionenstrahls dient und die Ionenstrahlteilchenzahl aufaddiert, bis die Dosis für eine Strahlposition erreicht ist, so daß dann ein Befehl an den Steuer- und Auslesemodul (SAMP) für die Pulszentrale gegeben werden kann, die über die Pulszentralansteuerung (PZA) die Weiterschaltung auf die nächste Strahlposition veranlaßt, die dann über die Echtzeit-Nachregelungsschleife an die Magnetstromnetzgeräte (MGN) des Rasterscanners weitervermittelt wird. Das Steuer- und Auslesemodul (SAMI1) ist schaltungs- und ablauftechnisch innerhalb der Ablaufsteuerung (VMEAS) vor dem Steuer- und Auslesemodul (SAMO1) des ortsempfindlichen Detektors (MWPC1) angeordnet. Dieser Datenflußplan der Figur 5 zeigt somit ein Ausführungsbeispiel der Erfindung.

Dabei sind innerhalb der Echtzeitsteuerung (VME-Crate) des Systems eine Reihe von Steuer- und Auslesemodulen (SAM) über Schnittstellen miteinander verbunden. Für das erfindungsgemäße Ausführungsbeispiel einer Regelungsanordnung sind die beiden SAMs relevant, welche den Ortsmeßdetektor 1 (SAMO1) steuern und auslesen und das Modul zur Speicherung der Meßdaten (SAMD). Die Regelschleife geht von der Vieldrahtionisationskammer (MWPC1) mit angeschlossenem Steuer- und Auslesemodul (SAMO1) zum Steuer- und Auslesemodul (SAMD), die dazwischenliegenden SAM-Module dienen lediglich dem Datentransfer von dem SAMO1 zum SAMD. Letzteres verfügt sowohl über genügend freie Schnittstellen als auch über genügend Rechenleistung, um die Nachführung der Ionenstrahlbreite auszuführen.

Generell besteht bei diesem modular aufgebauten Kontrollsystem auch die Möglichkeit, ein nicht gezeigtes zusätzliches Steuer- und Auslesemodul in die Steueranordnung zu integrieren, welches ausschließlich die Funktion der Breitenregelung erfüllt. Die Kapazität der dargestellten Anordnung reicht jedoch vollständig aus, um die Zusatzaufgabe einer Breitenregelung des Ionenstrahls in Zusammenwirken mit den Magnetnetzgeräten 18 und einer Regelschleife zwischen dem SAMD, dem SAMO1 und den Magnetnetzgeräten 18 der horizontalen und vertikalen Quadrupolmagneten 10 zu übernehmen.

### Bezugszeichenliste

- 1: Tumorgewebe
- 2: gesundes Gewebe
- 3: Rand des Tumorgewebes
- 4: Zentrum des Tumorgewebes
- 5: Ablaufsteuersystem
- 6: Systemkontrolle
- 7: Bedienerführung
- 8: Sicherheitssystem
- 9: Randbereich
- 10: Quadrupolmagnete zur Horizontal- und Vertikal-Fokussierung
- 11: Steuerungs- und Auslesemodul SAMI1
- 12: Steuerungs- und Auslesemodul SAMI2
- 13: Steuerungs- und Auslesemodul SAMP
- 14: Steuerungs- und Auslesemodul SAMO1
- 15: Steuerungs- und Auslesemodul SAMS
- 16: Steuerungs- und Auslesemodul SAMO2
- 17: Steuerungs- und Auslesemodul SAMD
- 18: Magnetnetzgeräte für horizontale und vertikale Quadrupole
- 19: Ionenstrahl
- 20: Rasterscanmagnete
- 27: ODS (Datenspeicher)
- 28: SKR (Systemkontrollrechner)
- 29: Beschleuniger Operating-Konsole im TKR
- 30: Beschleuniger-Ethernet
- 31: Technischer Kontrollraum Therapie, TKR
- 32: Ethernet-Router
- 33: Barcode-Leser, BCL
- 34: Therapie Operating-Rechner, TORT
- 35: Bedienungselemente Terminals
- 36: Technische Operating-Konsole Therapie
- 37: Medizin Bedienerkonsole, MBDK
- 38: Therapie Operating-Rechner, TORM
- 39: Ethernet-Bridge
- 40: Therapie-Ethernet
- 41: Analog-/Digital-IO Modul, ADIO
- 42: Kontrollbusadaptor, KBA
- 43: Interlockeinheit, ILE
- 44: Totmanneinheit, TME
- 45: Intensitätsmonitor, IMON
- 46: Ablaufsteuerungsrechner, ASR
- 47: Digital-IO-Modul, DIO
- 48: Therapie Cave M
- 49: getrennte Magnetnetzgeräteansteuerung zum Strahlabbruch des Beschleunigers (S02KQ1E und TH3MU1)
- 50: zur Pulszentrale des Beschleunigers, PZA
- 51: PET Kamera
- 52: Beschleunigerstrahlführung in das Cave M
- 53: Patientenliege
- 54: Mini-Ridge Filter
- 55: Ionisationskammer für Intensitätsmonitor
- 56: Vieldrahtkammer (Multi-Wire Proportional Chamber, MWPC)
- 57: Ionisationskammer zur Intensitätsmessung (IC)

## Patentansprüche

1. Vorrichtung zur Anpassung einer lonenstrahlfleckgröße, die nachfolgende Merkmals aufweist:
- eine Rasterscaneinrichtung aus Rasterscanmagneten (20) zur Rasterscannung des Ionenstrahls (19),
- Ionenstrahlfleckgröße bestimmende Quadrupolmagnete (10), die unmittelbar vor den Rasterscanmagneten (20) angeordnet sind,
- zwei Magnetstromnetzgeräte (18) für das Quadrupoldoublett der die Ionenstrahlfleckgröße bestimmenden Quadrupolmagnete (10), **dadurch gekennzeichnet, daß** die Vorrichtung
- eine Regelschleife (21) aufweist zum Bereitstellen von Stromkorrekturwerten unter Vergleich von Soll- und Istwerten einer aktuellen Strahlausdehnung für zwei Magnetstromnetzgeräte (18) des Quadrupoldoubletts der Ionenstrahlfleckgröße bestimmenden Quadrupolmagneten (10) zur definierten Homogenisierung und/oder zur definierten Variation der Ionenstrahlfleckgröße, während der Strahlextraktion und/oder von Meßzyklus zu Meßzyklus und/oder von Strahlposition zu Strahlposition,
und wobei die Vorrichtung ein Steuer- und Auslesemodul (SAMD) aufweist, das ein dichteres Ionenstrahlpositionsraster bei gleichzeitig geringerer Ionenstrahlfleckgröße im Randbereich (3) als im Volumenbereich (4) eines Tumors steuert und ausliest.

2. Vorrichtung nach Anspruch 1 **dadurch gekennzeichnet, daß** die Vorrichtung einen ortsempfindlichen Detektor (MWPC1) zur Erfassung der Ionenstrahlbreite und zur Erzeugung von Detektorrohdaten aufweist.

3. Vorrichtung nach einem der Ansprüche 1 bis 2, **dadurch gekennzeichnet, daß** die Vorrichtung mehrere Steuer- und Auslesemodule (SAM01 und SAMD) und zugehörige Datenverbindungen aufweist, um Informationen über den Istwert von Ionenstrahlbreiten an ein Speicher-, Steuer- und Auslesemodul (SAMD) zur Speicherung der Meßdaten zu schicken.

4. Vorrichtung nach einem der Ansprüche 1 bis 3, **dadurch gekennzeichnet, daß** ein Auslesemodul (SAMD) eine Anzahl freier Schnittstellen und eine Rechenleistung aufweist, über welche die Nachführung der Ionenstrahlbreite ausführbar ist.

5. Vorrichtung nach einem der Ansprüche 1 bis 4, **dadurch gekennzeichnet, daß** die Vorrichtung ein um ein Steuer- und Auslesemodul erweitertes Kontrollsystem aufweist, wobei durch das zusätzliche Steuer- und Auslesemodul ausschließlich die Funktion der Ionenstrahlbreitenregelung durchführbar ist.

6. Vorrichtung nach einem der Ansprüche 1 bis 5, **dadurch gekennzeichnet, daß** die Vorrichtung eine Regelschleife aufweist, die von einer Vieldrahtionisationskammer (MWPC1) mit angeschlossenem Steuer- und Auslesemodul (SAM01) ausgeht und zu einem Steuer- und Auslesemodul (SAMD) führt, das Meßdaten zur graphischen Darstellung bereitstellt und zwei Magnetstromnetzgeräte (18) zur horizontalen und vertikalen Fokussierung des Quadrupoldoubletts unmittelbar vor den Rasterscanmagneten (20) ansteuert.

## Claims

1. Apparatus for adapting the size of an ion beam spot, having the following features:
- a raster scanning device composed of raster scanning magnets (20) for raster scanning the ion beam (19),
- quadrupole magnets (10) determining the size of the ion beam spot, which quadrupole magnets (10) are arranged directly in front of the raster scanning magnets (20),
- two magnet power supply units (18) for the quadrupole doublet of the quadrupole magnets (10) determining the size of the ion beam spot, **characterized in that** the apparatus has
- a control loop (21) for providing current correction values, by comparing desired and actual values of the prevailing dimension of the beam, for two magnet power supply units (18) of the quadrupole doublet of the quadrupole magnets (10) determining the size of the ion beam spot, for defined homogenization and/or for defined variation of the size of the ion beam spot during beam extraction and/or from measuring cycle to measuring cycle and/or from beam position to beam position, and
- the apparatus having a control and read-out module (SAMD) that controls and reads out a denser ion beam position raster with, at the same time, a smaller ion beam spot size in the edge region (3) than in the volume region (4) of a tumour.

2. Apparatus according to claim 1, **characterized in that** the apparatus has a position-sensitive detector (MWPC1) for detecting the ion beam width and for producing raw detector data.

3. Apparatus according to any one of claims 1 to 2, **characterized in that** the apparatus has a plurality of control and read-out modules (SAM01 and SAMD) and associated data connections in order to send information on the actual value of ion beam widths to a storage, control and read-out module (SAMD) for storage of the measurement data.

4. Apparatus according to any one of claims 1 to 3, **characterized in that** one read-out module (SAMD) has a number of free interfaces and a computing capacity by means of which the tracking of the ion beam width can be performed.

5. Apparatus according to any one of claims 1 to 4, **characterized in that** the apparatus has a control system that is expanded by one control and read-out module, wherein exclusively the function of ion beam width regulation can be performed by the additional control and read-out module.

6. Apparatus according to any one of claims 1 to 5, **characterized in that** the apparatus has a control loop which starts from a multiwire ionization chamber (MWPC1) with attached control and read-out module (SAM01) and leads to a control and read-out module (SAMD) that provides measurement data for graphical representation and actuates two magnet power supply units (18) for horizontal and vertical focusing of the quadrupole doublet directly in front of the raster scanning magnets (20).

## Revendications

1. Dispositif pour l'adaptation de la taille des spots d'un faisceau ionique qui présente les caractéristiques suivantes :
- un dispositif de balayage vidéo constitué d'aimants de balayage vidéo (20) pour le balayage vidéo du faisceau ionique (19),
- des aimants quadripolaires (10) déterminant la taille des spots d'un faisceau ionique qui sont placés directement devant les aimants de balayage vidéo (20),
- deux unités d'alimentation magnétique (18) pour le doublet quadripolaire des aimants quadripolaires (10) déterminant la taille des spots d'un faisceau ionique, **caractérisé en ce que** le dispositif comporte une boucle de régulation (21) pour la fourniture de valeurs de correction de courant par comparaison entre des valeurs théoriques et des valeurs réelles d'un élargissement actuel d'un faisceau pour deux unités d'alimentation magnétique (18) du doublet quadripolaire des aimants quadripolaires (10) déterminant la taille des spots d'un faisceau ionique pour l'homogénéisation définie et/ou pour la variation définie de la taille des spots d'un faisceau ionique pendant l'extraction du faisceau et/ou d'un cycle de mesure à l'autre et/ou d'une position de faisceau à l'autre, et dans lequel le dispositif comporte un module de commande et de lecture (SAMD)qui commande et qui lit une trame de position de faisceau ionique plus dense avec, en même temps, une taille de spots du faisceau ionique plus petite dans la zone marginale (3) que dans la zone volumique (4) d'une tumeur.

2. Dispositif selon la revendication 1, **caractérisé en ce que** le dispositif comporte un détecteur sensible localement (MWPC1) pour la détection de la largeur du faisceau ionique et pour la génération de données brutes de détecteur.

3. Dispositif selon l'une quelconque des revendications 1 à 2, **caractérisé en ce que** le dispositif comporte plusieurs modules de commande et de lecture (SAM01 et SAMD) et des circuits de données associés pour envoyer des informations sur la valeur réelle des largeurs du faisceau ionique à un module de mémoire, de commande et de lecture (SAMD) pour la mémorisation des données de mesure.

4. Dispositif selon l'une quelconque des revendications 1 à 3, **caractérisé en ce qu'**un module de lecture (SAMD) présente un certain nombre d'interfaces libres et une puissance de calcul au moyen desquels on peut réaliser la recopie de la largeur du faisceau ionique.

5. Dispositif selon l'une quelconque des revendications 1 à 4, **caractérisé en ce que** le dispositif comporte un système de contrôle augmenté par un module de commande et de lecture, dans lequel la fonction de régulation de la largeur du faisceau ionique est réalisable exclusivement au moyen du module de commande et de lecture supplémentaire.

6. Dispositif selon l'une quelconque des revendications 1 à 5, **caractérisé en ce que** le dispositif comporte une boucle de régulation qui part d'une chambre d'ionisation à plusieurs fils (MWPC1) avec un module de commande et de lecture raccordé (SAM01) et qui conduit à un module de commande et de lecture (SAMD) qui fournit des données de mesure pour la représentation graphique et qui commande deux unités d'alimentation magnétique (18) pour la focalisation horizontale et verticale du doublet quadripolaire directement devant les aimants de balayage vidéo (20).
